# EUROPÄISCHE PATENTANMELDUNG

(11) **EP 1 908 546 A1**
(43) Veröffentlichungstag der Anmeldung: **09.04.2008**
(21) Anmeldenummer: 06020907.9
(22) Anmeldetag: 05.10.2006
(51) Int. Cl.: B23K 26/20, B23K 26/36, B23K 26/42, B23K 37/00, A61B 19/00, A61F 9/02, F16P 1/00, F16P 3/06, G02B 5/26

(54) **Diffus reflektierend ausgebildeter Laserstrahlreflektor ; Arbeitsraumabtrennung sowie Arbeitsraum mit Laser-Bearbeitungseinrichtung mit einem solchen Laserstrahlreflektor**

(71) Anmelder: Trumpf Laser- und Systemtechnik GmbH, 71254 Ditzingen (DE)
(72) Erfinder: Pieger, Markus, 70191 Stuttgart (DE)
(74) Vertreter: Kohler Schmid Möbus

(57) **Zusammenfassung**

Ein Arbeitsraum (1) mit einer Laser-Bearbeitungseinrichtung (3) im Arbeitsrauminnern (2) ist mittels einer Arbeitsraumabtrennung (7) gegen die Arbeitsraumumgebung (8) abgeschirmt. Die Arbeitsraumabtrennung (7) weist an ihrer der Laser-Bearbeitungseinrichtung (3) zugewandten Innenseite einen Laserstrahlreflektor (12) auf, mittels dessen der überwiegende Teil von an dem Laserstrahlreflektor (12) einfallender und von der Laser-Bearbeitungseinrichtung (3) stammender Laserstrahlung, reflektierbar ist. Der Laserstrahlreflektor (12) ist dabei diffus reflektierend ausgebildet.
Eine Arbeitsraumabtrennung (7) für den genannten Arbeitsraum (1) sowie ein Laserstrahlreflektor (12) für eine derartige Arbeitsraumabtrennung (7) sind entsprechend ausgeführt.

## Beschreibung

Die Erfindung betrifft einen Arbeitsraum mit einer Laser-Bearbeitungseinrichtung im Arbeitsrauminnern sowie mit einer Arbeitsraumabtrennung zwischen dem Arbeitsrauminnern und der Arbeitsraumumgebung. Die Erfindung betrifft des Weiteren eine Arbeitsraumabtrennung der genannten Art sowie einen Laserstrahlreflektor hierfür.

Arbeitsräume, in denen Laser-Bearbeitungseinrichtungen betrieben werden, sind insbesondere aus Gründen des Arbeitsschutzes gegen ihre Umgebung abzuschirmen. Gemäß den einschlägigen Sicherheitsvorschriften muss gewährleistet sein, dass unter anderem auch bei irregulären Betriebszuständen der Laser-Bearbeitungseinrichtung und zumindest für eine begrenzte Zeitdauer keine schädliche Laserstrahlung aus dem Arbeitsrauminnern in die Arbeitsraumumgebung austreten kann. Zu diesem Zweck dienen Arbeitsraumabtrennungen, so genannte "Laserschutzwände".

Ein gattungsgemäßer Arbeitsraum sowie eine gattungsgemäße Arbeitsraumabtrennung sind offenbart in US 4,650,287 A. Im Falle des Standes der Technik wird ein Arbeitsraum, der eine Laserbearbeitungsmaschine beherbergt, durch eine strahlungsabsorbierende Laserschutzwand eingefasst. Bei der vorbekannten Laserschutzwand handelt es sich um eine Verbundkonstruktion aus einem Metallblech an der von der Laserbearbeitungsmaschine abliegenden Wandaußenseite sowie aus einer strahlungsabsorbierenden Beschichtung, die auf das Metallblech an dessen zu der Laserbearbeitungsmaschine hin gelegenen Innenseite aufgetragen ist. Gebildet wird die strahlungsabsorbierende Beschichtung durch Metalloxide, beispielsweise durch Aluminium-, Titan-, Zirkon-, Magnesium- und/oder Chromoxid. Die Fähigkeit dieser Metalloxid-Beschichtungen zur Absorption von Laserstrahlung wird in US 4,650,287 A als außerordentlich bezeichnet.

Die aus US 4,650,287 A bekannte Laserschutzwand wirkt demzufolge als Strahlungsfalle, die als solche den überwiegenden Teil der Energie der am Verlassen des Arbeitsrauminnern zu hindernden Laserstrahlung aufnimmt. Folgerichtig wird in der Vorveröffentlichung die besondere Hitzebeständigkeit der strahlungsabsorbierenden Metalloxid-Beschichtungen hervorgehoben.

Das Ausmaß der in die vorbekannte Laserschutzwand eingekoppelten Strahlungsenergie wirkt sich negativ auf die Standzeit der Laserschutzwand unter Strahlungsbelastung aus. Dies wiederum beeinträchtigt die Eignung der vorbekannten Laserschutzwand als Einrichtung des Arbeitsschutzes.

Die Aufgabe der vorliegenden Erfindung besteht darin, an einem Arbeitsraum der eingangs genannten Art maximalen Arbeitsschutz in Kombination mit maximaler Betriebssicherheit der in dem Arbeitsraum untergebrachten Laser-Bearbeitungseinrichtung zu gewährleisten.

Erfindungsgemäß gelöst wird diese Aufgabe durch den Arbeitsraum gemäß Patentanspruch 1, durch die Arbeitsraumabtrennung gemäß Patentanspruch 16 sowie durch den Laserstrahlreflektor gemäß Patentanspruch 17.

Im Falle der Erfindung ist an der Innenseite der Arbeitsraumabtrennung ein Laserstrahlreflektor vorgesehen, welcher den überwiegenden Teil der an dem Verlassen des Arbeitsrauminnern zu hindernden Laserstrahlung reflektiert. Der hohe Reflexionsgrad des erfindungsgemäßen Laserstrahlreflektors verhindert, dass Strahlungsenergie der an dem Laserstrahlreflektor einfallenden Laserstrahlung in einem Ausmaß in die Arbeitsraumabtrennung eingekoppelt wird, das bereits nach kurzer Zeit zu einer Überhitzung und einer damit verbundenen Zerstörung der Arbeitsraumabtrennung führen würde. Insofern gewährleisten die Merkmale der Erfindung einen maximalen Arbeitsschutz.

Gleichzeitig wird die einfallende Laserstrahlung von dem erfindungsgemäßen Laserstrahlreflektor nicht gerichtet sondern vielmehr diffus reflektiert. In Folge ihrer Streuung besitzt die reflektierte Laserstrahlung eine lediglich geringe Energiedichte und ist daher nicht in der Lage, im Arbeitsrauminnern, insbesondere an der dort angeordneten Laser-Bearbeitungseinrichtung Schaden anzurichten. Insofern sorgt die Erfindung für eine Maximierung der Betriebssicherheit der Laser-Bearbeitungseinrichtung. Ansonsten zu verwendende Schutzeinrichtungen an der Laser-Bearbeitungseinrichtung, beispielsweise Schutzhauben für Faltenbälge, sind verzichtbar.

Sowohl unter dem Gesichtspunkt des Arbeitsschutzes als auch unter dem Aspekt der Betriebssicherheit der Laser-Bearbeitungseinrichtung sind die erfindungsgemäßen Merkmale dann von besonderem Vorteil, wenn an der Laser-Bearbeitungseinrichtung mit Laserstrahlung hoher Leistung gearbeitet wird.

Besondere Ausführungsarten der Erfindung gemäß den Patentansprüchen 1, 16 und 17 ergeben sich aus den abhängigen Patentansprüchen 2 bis 15.

Die Erfindungsbauart nach Patentanspruch 2 zeichnet sich dadurch aus, dass der Laserstrahlreflektor materialbezogen auf die Wellenlänge der zu reflektierenden Laserstrahlung abgestimmt ist. Auf diese Art und Weise ist sichergestellt, dass auch tatsächlich der überwiegende Teil der an dem Laserstrahlreflektor einfallenden Laserstrahlung reflektiert wird. Die Wellenlänge des zu reflektierenden Laserlichtes ist abhängig von der Bauart der Strahlquelle. Etwa an einem Laserstrahlreflektor aus Aluminium kann CO₂-Laserlicht zu 99%, NdYAG-Laserlicht zu 90% reflektiert werden. Generell werden erfindungsgemäß Laserstrahlreflektoren mit einem Reflexionsgrad von wenigstens 90% bevorzugt. Bei einem derartigen Reflexionsgrad des Laserstrahlreflektors lassen sich hinreichende Standzeiten der Arbeitsraumabtrennung gewährleisten.

Besonders praxistauglich sind Laserstrahlreflektoren in Form diffus reflektierender metallischer Oberflächen (Patentanspruch 3). Derartige Laserstrahlreflektoren lassen sich verhältnismäßig einfach fertigen und zeigen aufgrund ihrer Robustheit auch unter rauen Einsatzbedingungen und über eine lange Einsatzdauer die erforderliche Reflexionsfähigkeit.

Als Werkstoffe für als Laserstrahlreflektoren dienende metallische Oberflächen kommen erfindungsgemäß verschiedene Metalle in Frage (Patentanspruch 4). Als Laserstrahlreflektor bevorzugt wird im Falle der Erfindung eine diffus reflektierende Aluminiumoberfläche. Derartige Laserstrahlreflektoren sind insbesondere kostengünstig. Außerdem empfiehlt sich die Verwendung von Aluminium als Reflektorwerkstoff aufgrund seiner (beispielsweise verglichen mit Kupfer) verhältnismäßig geringen Wärmeleitfähigkeit und aufgrund seines geringen Gewichts. Schließlich lässt sich mit Aluminium - wie vorstehend zu Patentanspruch 2 dargelegt - bei unterschiedlichen Wellenlängen des zu reflektierenden Laserlichtes stets ein hoher Reflexionsgrad erreichen.

Der Einfachheit halber, insbesondere im Interesse einer möglichst einfachen und kostengünstigen Fertigung, wird im Falle der Erfindung als den Laserstrahlreflektor bildende diffus reflektierende Oberfläche eine blanke Oberfläche gegebenenfalls eine blanke metallische Oberfläche, bevorzugt.

Der Laserstrahlreflektor, gegebenenfalls die den Laserstrahlreflektor bildende metallische Oberfläche, kann von einer auf einem Träger aufgebrachten Beschichtung gebildet sein (Patentanspruch 6). Als Beschichtungsverfahren kommen beispielsweise das Flammspritzen oder Metall-Abscheideverfahren in Frage.

Im Interesse einer besonders einfachen und kostengünstigen Fertigung empfiehlt es sich, als Laserstrahlreflektor unmittelbar eine Oberfläche eines Reflektorkörpers vorzusehen (Patentanspruch 7). Das Aufbringen einer als Laserstrahlreflektor dienenden Oberflächenbeschichtung auf einen Träger erübrigt sich in diesem Fall.

In bevorzugter Ausgestaltung der Erfindung ist als mit seiner Oberfläche den Laserstrahlreflektor bildender Reflektorkörper ein Metallkörper, insbesondere ein Metallblech, vorgesehen (Patentanspruch 8). Erfindungsgemäß bevorzugt wird ein Aluminiumblech, das mit einer blanken und diffus reflektierenden Aluminiumoberfläche den Laserstrahlreflektor bildet.

Eine diffuse Reflexion der einfallenden Laserstrahlung durch den Laserstrahlreflektor kann erfindungsgemäß auf unterschiedliche Art und Weise bewirkt werden.

Aus fertigungstechnischen Gründen ist es empfehlenswert, den Laserstrahlreflektor mit einer entsprechenden räumlichen Oberflächenstruktur zu versehen (Patentanspruch 9). Dabei lässt sich eine besonders intensive Streuung des reflektierten Laserlichtes mittels unregelmäßig profilierter Oberflächenstrukturen erzielen (Patentanspruch 10). Gleichwohl sind aber auch Laserstrahlreflektoren mit regelmäßig profilierter Oberflächenstruktur denkbar. Beispielsweise durch prägende Bearbeitung lassen sich insbesondere an Blechen, die unmittelbar mit ihrer Oberfläche den Laserstrahlreflektor bilden, erfindungsgemäße Oberflächenstrukturen erzeugen.

Handelsüblich und somit vorteilhafterweise frei verfügbar sind genarbte Oberflächenstrukturen (Patentanspruch 11).

Eine optimale Funktionsfähigkeit erfindungsgemäßer Laserstrahlreflektoren setzt deren Formstabilität voraus. Zur Gewährleistung dieser Formstabilität sind im Falle der Erfindungsbauart nach Patentanspruch 12 versteifende Verformungen an dem mit einer diffus reflektierenden Beschichtung versehenen Träger bzw. an dem eine diffus reflektierende Oberfläche ausbildenden Reflektorkörper vorgesehen. Als handelsübliche und somit ohne weiteres erhältliche Träger bzw. Reflektorkörper bieten sich Trapezbleche an (Patentanspruch 13). Versteifungen der genannten Art sind insbesondere dann zweckmäßig, wenn der Laserstrahlreflektor im Deckenbereich des Arbeitsraumes bzw. der Arbeitsraumabtrennung vorgesehen ist.

Gemäß Patentanspruch 14 ist ein Laserstrahlreflektor Teil einer Arbeitsraumabtrennung, die außerdem eine Außenlage umfasst, die ihrerseits an der von der Laser-Bearbeitungseinrichtung abgewandten Seite des Laserstrahlreflektors angeordnet ist. Zweckmäßigerweise ist der Laserstrahlreflektor dabei mit der Außenlage der Arbeitsraumabtrennung lösbar verbunden. Aufgrund dieser lösbaren Verbindung lässt sich beispielsweise ein defekter bzw. verschlissener Laserstrahlreflektor auf einfache Art und Weise gegen einen voll funktionsfähigen Laserstrahlreflektor austauschen. Auch lässt sich ein Laserstrahlreflektor etwa zur Durchführung von Wartungs-, insbesondere von Reinigungsarbeiten, problemlos von der zugehörigen Außenlage der Arbeitsraumabtrennung abnehmen und nach der Wartung bzw. Reinigung wieder an der Außenlage anbringen.

Im Interesse einer thermischen Isolierung der Außenlage der Arbeitsraumabtrennung gegen den Laserstrahlreflektor verbleibt im Falle der Erfindungsbauart nach Patentanspruch 15 zwischen dem Laserstrahlreflektor und der Außenlage der Arbeitsraumabtrennung ein Zwischenraum.

Nachstehend wird die Erfindung anhand beispielhafter und stark schematisierter Darstellungen näher erläutert. Es zeigen:
- Figur 1: einen Arbeitsraum in Form einer Laserschutzkabine mit einer Laser-Bearbeitungseinrichtung und einer Arbeitsraumabtrennung,
- Figur 2: das Detail II von Figur 1,
- Figur 3: das Detail III von Figur 1,
- Figur 4: einen Teilbereich der Arbeitsraumabtrennung gemäß Figur 1 in der perspektivischen Ansicht vom Arbeitsrauminnern her,
- Figur 5: eine Schnittdarstellung der Arbeitsraumabtrennung gemäß Figur 1 und
- Figur 6: eine Schnittdarstellung einer zweiten Bauart einer Arbeitsraumabtrennung.

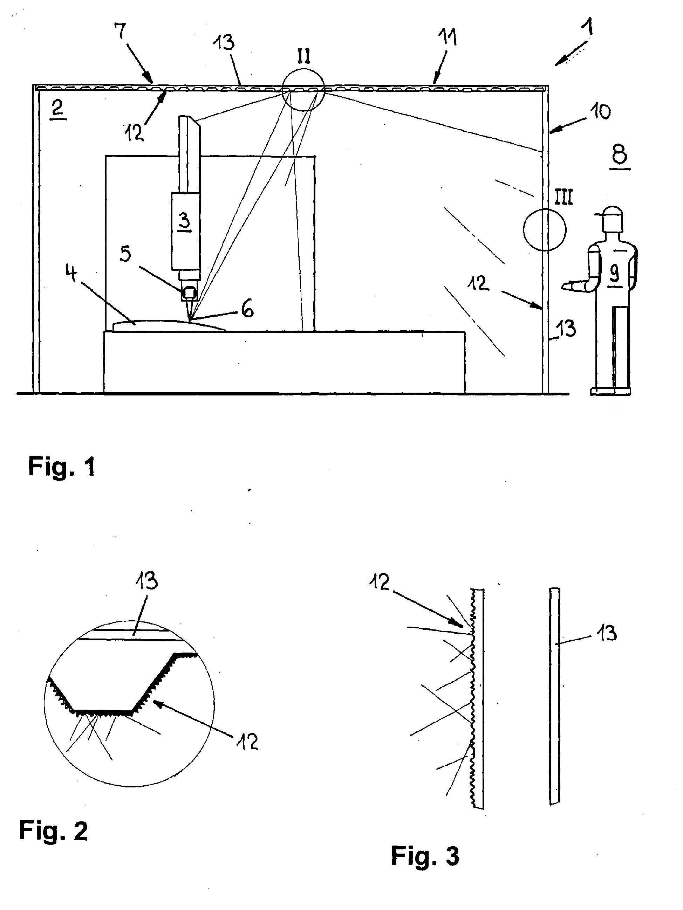

Gemäß Figur 1 beherbergt ein als Laserschutzkabine ausgebildeter Arbeitsraum 1 im Arbeitsrauminnern 2 eine Laser-Bearbeitungseinrichtung 3, die im dargestellten Beispielsfall als Laserschneideinrichtung für die 3D-Bearbeitung von Werkstücken 4 ausgeführt ist. Von einem Laserschneidkopf 5 der Laser-Bearbeitungseinrichtung 3 aus wird im regulären Schneidbetrieb ein Laserstrahl 6 auf das zu bearbeitende Werkstück 4 gerichtet.

Wie in Figur 1 angedeutet, wird der Laserstrahl 6 an der Oberfläche des Werkstückes 4 teilweise reflektiert. Die von der Oberfläche des Werkstückes 4 abgestrahlte Laserstrahlung trifft auf die Innenseite einer Arbeitsraumabtrennung 7. Diese umgibt die Laser-Bearbeitungseinrichtung 3 haubenartig und schirmt das Arbeitsrauminnere 2 gegen die Arbeitsraumumgebung 8 ab. In der Arbeitsraumumgebung 8 hält sich eine Bedienperson 9 auf.

Die Arbeitsraumabtrennung 7 umfasst einen vertikalen Wandbereich 10 sowie einen horizontalen Deckenbereich 11. Sowohl in dem Wandbereich 10 als auch in dem Deckenbereich 11 weist die Arbeitsraumabtrennung 7 an ihrer der Laser-Bearbeitungseinheit 3 zugewandten Innenseite einen Laserstrahlreflektor 12 auf. Der Laserstrahlreflektor 12 wird gebildet von der Oberfläche von Zuschnitten aus Aluminiumblech mit einer Dicke von 1 mm. In dem Deckenbereich 11 der Arbeitsraumabtrennung 7 sind als Blechzuschnitte Trapezbleche vorgesehen. Sowohl in dem Wandbereich 10 als auch in dem Deckenbereich 11 der Arbeitsraumabtrennung 7 sind die Blechzuschnitte mit einer Außenlage bzw. Außenschale 13 der Arbeitsraumabtrennung 7 verschraubt. Die Außenschale 13 der Arbeitsraumabtrennung 7 ist aus zwei Millimeter dickem Stahlblech gefertigt.

Das Aluminiumblech des Laserstrahlreflektors 12 ist handelsüblich. Etwa im Baubereich wird es unter anderem als Fassaden- oder Dachverkleidung verwendet. Die Blechoberfläche, welche den Laserstrahlreflektor 12 ausbildet, ist blank und besitzt eine räumliche Oberflächenstruktur, die in den Figuren 2 und 3 andeutungsweise gezeigt ist. In Figur 1 ist der Laserstrahlreflektor 12 der Einfachheit halber als ebene Oberfläche dargestellt.

Die räumliche Oberflächenstruktur des für den Laserstrahlreflektor 12 verwendeten Aluminiumblechs ist unregelmäßig und entspricht in etwa der Oberflächenstruktur von genarbtem Leder. Im Handel ist diese Oberflächenstruktur unter der Bezeichnung "stucco dessiniert" erhältlich. Alternativ wäre unter anderem auch der Einsatz von Aluminiumblech mit regelmäßig strukturierter Oberfläche, beispielsweise mit sphärischen Strukturen denkbar.

Ausschlaggebend ist das Reflexionsverhalten der Blechoberfläche. Zum einen muss gewährleistet sein, dass die von der Laser-Bearbeitungseinrichtung 3 stammende Laserstrahlung an der den Laserstrahlreflektor 12 bildenden Blechoberfläche zum überwiegenden Teil reflektiert wird. Diese Voraussetzung wird von der den Laserstrahlreflektor 12 bildenden blanken Aluminiumoberfläche des gezeigten Ausführungsbeispiels in hohem Maße erfüllt. So reflektiert der Laserstrahlreflektor 12 rund 99% der an ihm einfallenden Laserstrahlung. Erreicht wird dieser außerordentlich hohe Reflexionsgrad bei der Wellenlänge des in dem gezeigten Beispielfall auf den Laserstrahlreflektor 12 treffenden Laserlichtes. Die Wellenlänge des Laserlichtes wiederum ist abhängig von der Bauart der Strahlquelle. Zur Erzeugung des Laserstrahles 6 wird ein CO₂-Generator eingesetzt. Würde das an dem Laserstrahlreflektor 12 einfallende Laserlicht mittels eines NdYAG-Lasers erzeugt, so ließe sich mit der blanken Aluminiumoberfläche des Ausführungsbeispiels ein Reflexionsgrad von rund 90% erzielen. Aufgrund der gewählten Oberflächenstruktur des Laserstrahlreflektors 12 wird ein Reflexionsgrad in der genannten Größenordnung selbst bei einer im Nahbereich von Laserschneideinrichtungen üblichen Verschmutzung des Laserstrahlreflektors 12 erreicht.

Aufgrund des hohen Reflexionsvermögens des Laserstrahlreflektors 12 kommt es unter der Wirkung des an dem Laserstrahlreflektor 12 einfallenden Laserlichtes zu einer lediglich geringen Erwärmung des Laserstrahlreflektors 12 und somit der gesamten Arbeitsraumabtrennung 7. Die Arbeitsraumabtrennung 7 erreicht daher lange Standzeiten.

Gleichzeitig sorgt die Oberflächenstruktur des Laserstrahlreflektors 12 für eine diffuse Reflexion des einfallenden Laserlichtes, wie sie in den Figuren 2 und 3 angedeutet ist. Die Energiedichten in dem von dem Laserstrahlreflektor 12 abgestrahlten Laserlicht liegen daher auf einem lediglich niedrigen Niveau. Insbesondere sind die genannten Energiedichten zu gering, als dass sie in dem Arbeitsrauminnern 2, insbesondere an der Laser-Bearbeitungseinrichtung 3 Schäden verursachen könnten. Folglich kann beispielsweise auf ansonsten benötigte Schutzhauben für Faltenbälge der Laser-Bearbeitungseinrichtung 3 verzichtet werden.

Die beschriebene Schutzwirkung nach innen und nach außen entfaltet der Laserstrahlreflektor 12 auch bei irregulären Betriebszuständen der Laser-Bearbeitungseinrichtung 3, etwa dann, wenn der Laserstrahl 6 von dem Laserschneidkopf 5 versehentlich auf den Laserstrahlreflektor 12 gerichtet wird.

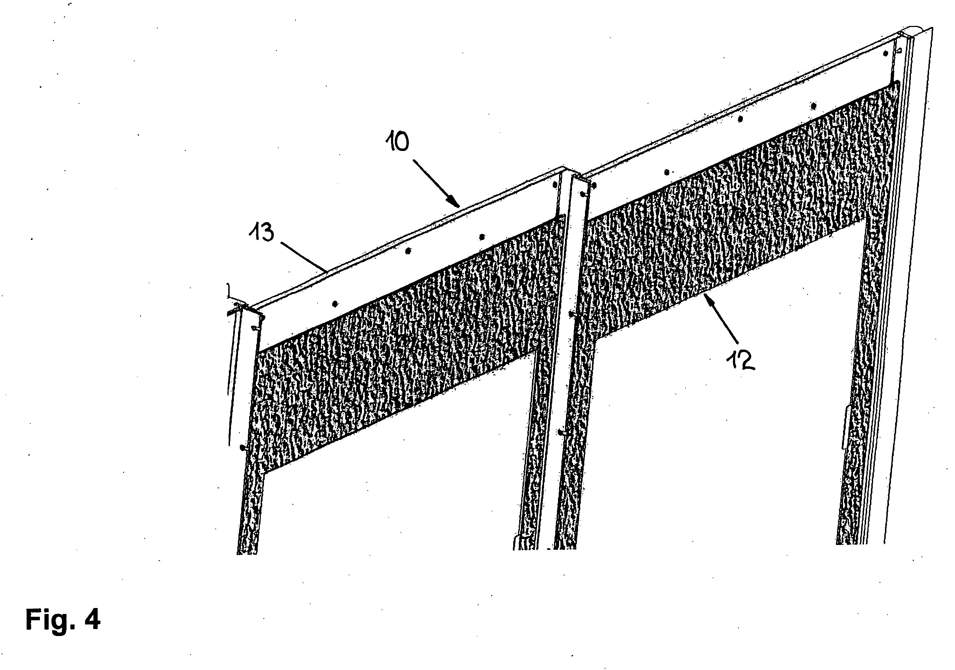

Figur 4 zeigt den Laserstrahlreflektor 12 an der Außenschale 13 der Arbeitsraumabtrennung 7 in der Ansicht von Arbeitsrauminnern 2 her. Der dargestellte Wandbereich ist mit Fensterausschnitten versehen. Deutlich zu erkennen ist die Oberflächenstruktur des Laserstrahlreflektors 12.

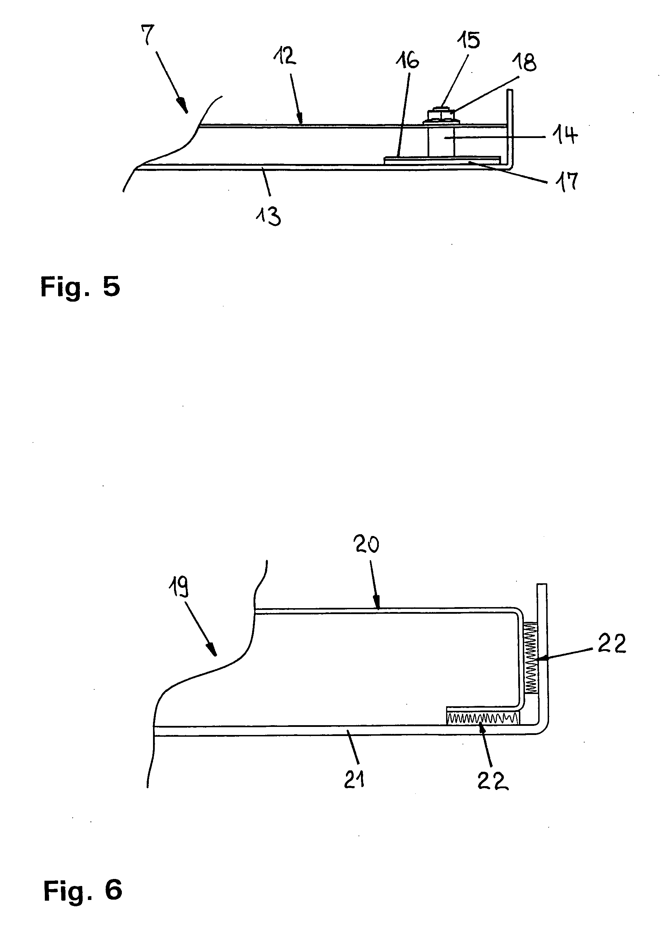

Figur 5 zeigt eine Schraubverbindung zwischen dem Laserstrahlreflektor 12 und der Außenschale 13 der Arbeitsraumabtrennung 7 im Detail.

Demnach wird der Laserstrahlreflektor 12 mittels einer Distanzhülse 14 auf Abstand von der Außenschale 13 gehalten. Der sich damit ergebende Zwischenraum zwischen dem Laserstrahlreflektor 12 und der Außenschale 13 der Arbeitsraumabtrennung 7 dient der thermischen Isolierung der Außenschale 13 gegen den durch Laserstrahlung beaufschlagten Laserstrahlreflektor 12. Die Distanzhülse 14 wird von einem Schraubenbolzen 15 durchsetzt, der an seinem der Außenschale 13 zugeordneten Längsende mit einer Fußplatte 16 verschweißt ist. Die Fußplatte 16 ist auf einen beidseitig selbstklebenden Zuschnitt 17 aus Moosgummi aufgeklebt. Der Zuschnitt 17 wiederum ist an seiner von der Fußplatte 16 abliegenden Seite mit der Außenschale 13 verklebt. An dem freien Ende des Schraubenbolzens 15 ist der Laserstrahlreflektor 12 zwischen einer Mutter 18 und der Distanzhülse 14 verspannt. Entsprechende Verbindungen zwischen dem Laserstrahlreflektor 12 und der Außenschale 13 der Arbeitsraumabtrennung 7 sind an den übrigen Befestigungsstellen des Laserstrahlreflektors 12 hergestellt.

Zur Demontage des Laserstrahlreflektors 12 sind entweder die Muttern 18 oder die Klebverbindungen zwischen den Zuschnitten 17 der einzelnen Befestigungseinrichtungen und der Außenschale 13 zu lösen. Zur Neumontage des Laserstrahlreflektors 12 werden in dem letztgenannten Fall die alten Zuschnitte 17 an den Fußplatten 16 der verschiedenen Befestigungsvorrichtungen durch neue Zuschnitte mit noch unbenutzten Klebeflächen ersetzt. Anschließend lässt sich der Laserstrahlreflektor 12 an den auf die Fußplatten 16 aufgebrachten neuen Zuschnitten 17 auf die Innenseite der Außenschale 13 der Arbeitsraumabtrennung 7 aufkleben.

Figur 6 zeigt eine Arbeitsraumabtrennung 19 mit einem Laserstrahlreflektor 20 und einer Außenlage bzw. Außenschale 21. Der Laserstrahlreflektor 20 ist an seinem Rand U-förmig abgekantet. Zwischen zwei Schenkeln der U-förmigen Abkantung des Laserstrahlreflektors 20 und der Außenschale 21 ist jeweils eine Klettverbindung 22 hergestellt. Zu diesem Zweck sind einerseits an dem Laserstrahlreflektor 20 und andererseits an der Außenschale 21 herkömmliche Klettbandabschnitte angebracht. Die Klettverbindungen 22 gestatten eine handhabungsfreundliche Montage und Demontage des Laserstrahlreflektors 20.

Sowohl der Laserstrahlreflektor 12 gemäß Figur 5 als auch der Laserstrahlreflektor 20 gemäß Figur 6 wird von einer "stucco dessinierten" Oberfläche von Zuschnitten aus Aluminiumblech gebildet. Von einer detaillierten Darstellung der genannten Oberflächenstruktur wurde in den Figuren 5 und 6 aus Gründen der Darstellungsvereinfachung abgesehen.

## Patentansprüche

1. Arbeitsraum mit einer Laser-Bearbeitungseinrichtung (3) im Arbeitsrauminnern (2) sowie mit einer Arbeitsraumabtrennung (7, 19) zwischen dem Arbeitsrauminnern (2) und der Arbeitsraumumgebung (8), **dadurch gekennzeichnet, dass** die Arbeitsraumabtrennung (7, 19) an ihrer der Laser-Bearbeitungseinrichtung (3) zugewandten Innenseite einen Laserstrahlreflektor (12, 20) aufweist, mittels dessen der überwiegende Teil von an dem Laserstrahlreflektor (12, 20) einfallender und von der Laser-Bearbeitungseinrichtung (3) stammender Laserstrahlung reflektierbar ist, wobei der Laserstrahlreflektor (12, 20) diffus reflektierend ausgebildet ist.

2. Arbeitsraum nach Anspruch 1, **dadurch gekennzeichnet, dass** der Laserstrahlreflektor (12, 20) bezüglich seines Werkstoffs auf die Wellenlänge der zu reflektierenden Laserstrahlung abgestimmt ist.

3. Arbeitsraum nach einem der vorhergehenden Ansprüche, **dadurch gekennzeichnet, dass** als Laserstrahlreflektor (12, 20) eine metallische Oberfläche vorgesehen ist, die diffus reflektierend ausgebildet ist.

4. Arbeitsraum nach einem der vorhergehenden Ansprüche, **dadurch gekennzeichnet, dass** als den Laserstrahlreflektor (12, 20) bildende metallische Oberfläche eine Aluminium- und/oder eine Kupfer- und/oder eine Molybdän- und/oder eine Chrom- und/oder eine Silber- und/oder eine Goldoberfläche vorgesehen ist, die diffus reflektierend ausgebildet ist.

5. Arbeitsraum nach einem der vorhergehenden Ansprüche, **dadurch gekennzeichnet, dass** als den Laserstrahlreflektor (12, 20) bildende Oberfläche, gegebenenfalls als den Laserstrahlreflektor (12, 20) bildende metallische Oberfläche, eine blanke Oberfläche, gegebenenfalls eine blanke metallische Oberfläche, vorgesehen ist, die diffus reflektierend ausgebildet ist.

6. Arbeitsraum nach einem der vorhergehenden Ansprüche, **dadurch gekennzeichnet, dass** der Laserstrahlreflektor (12, 20), gegebenenfalls die den Laserstrahlreflektor (12, 20) bildende metallische Oberfläche, von einer auf einen Träger aufgebrachten Beschichtung gebildet ist.

7. Arbeitsraum nach einem der vorhergehenden Ansprüche, **dadurch gekennzeichnet, dass** als Laserstrahlreflektor (12, 20) unmittelbar eine Oberfläche eines Reflektorkörpers vorgesehen ist.

8. Arbeitsraum nach einem der vorhergehenden Ansprüche, **dadurch gekennzeichnet, dass** als unmittelbar mit seiner Oberfläche den Laserstrahlreflektor bildender Reflektorkörper ein Metallkörper, insbesondere ein Metallblech, vorgesehen ist.

9. Arbeitsraum nach einem der vorhergehenden Ansprüche, **dadurch gekennzeichnet, dass** der Laserstrahlreflektor (12, 20) aufgrund seiner räumlichen Oberflächenstruktur diffus reflektierend ausgebildet ist.

10. Arbeitsraum nach einem der vorhergehenden Ansprüche, **dadurch gekennzeichnet, dass** als diffus reflektierende räumliche Oberflächenstruktur des Laserstrahlreflektors (12, 20) eine unregelmäßig profilierte Oberflächenstruktur vorgesehen ist.

11. Arbeitsraum nach einem der vorhergehenden Ansprüche, **dadurch gekennzeichnet, dass** als diffus reflektierende räumliche Oberflächenstruktur des Laserstrahlreflektors (12, 20) eine genarbte Oberflächenstruktur vorgesehen ist.

12. Arbeitsraum nach einem der vorhergehenden Ansprüche, **dadurch gekennzeichnet, dass** der Träger, welcher mit der den Laserstrahlreflektor (12, 20) bildenden Beschichtung versehen ist, oder der Reflektorkörper, der den Laserstrahlreflektor (12, 20) unmittelbar mit seiner Oberfläche bildet, wenigstens teilweise eine versteifende Verformung aufweist.

13. Arbeitsraum nach einem der vorhergehenden Ansprüche, **dadurch gekennzeichnet, dass** der Träger, welcher mit der den Laserstrahlreflektor (12, 20) bildenden Beschichtung versehen ist, oder der Reflektorkörper, der den Laserstrahlreflektor (12, 20) unmittelbar mit seiner Oberfläche bildet, wenigstens teilweise als Trapezblech ausgebildet ist.

14. Arbeitsraum nach einem der vorhergehenden Ansprüche, **dadurch gekennzeichnet, dass** der Laserstrahlreflektor (12, 20) mit einer Außenlage (13, 21) der Arbeitsraumabtrennung (7, 19) lösbar verbunden ist, wobei die Außenlage (13, 21) der Arbeitsraumabtrennung (7, 19) an der von der Laser-Bearbeitungseinrichtung (3) abgewandten Seite des Laserstrahlreflektors (12, 20) angeordnet ist.

15. Arbeitsraum nach einem der vorhergehenden Ansprüche, **dadurch gekennzeichnet, dass** der Laserstrahlreflektor (12, 20) unter Ausbildung eines Zwischenraums zwischen dem Laserstrahlreflektor (12, 20) und der Außenlage (13, 21) der Arbeitsraumabtrennung (7, 19) mit der Außenlage (13, 21) der Arbeitsraumabtrennung (7, 19) verbunden ist.

16. Arbeitsraumabtrennung für einen Arbeitsraum (1) mit einer Laser-Bearbeitungseinrichtung (3), **gekennzeichnet durch** die auf die Arbeitsraumabtrennung (7, 19) bezogenen Merkmale wenigstens eines des vorhergehenden Ansprüche.

17. Laserstrahlreflektor für eine Arbeitsraumabtrennung (7, 19) eines Arbeitsraumes (1) mit einer Laser-Bearbeitungseinrichtung (3), **gekennzeichnet durch** die auf den Laserstrahlreflektor (12, 20) bezogenen Merkmale wenigstens eines der Ansprüche 1 bis 15.
